# EUROPEAN PATENT SPECIFICATION

(11) **EP 4 294 373 B1**
(45) Date of publication and mention of the grant of the patent: **25.03.2026**
(21) Application number: 22756623.9
(22) Date of filing: 03.02.2022
(51) Int. Cl.: A61L 27/56, A61L 27/54, A61L 27/24, A61L 27/20, A61L 27/18, A61L 27/38, A61K 47/42, A61L 27/14, A61K 47/36, A61K 9/00, A61K 47/34

(54) **A METHOD FOR OBTAINING INJECTABLE BIOCOMPATIBLE DRUG DELIVERY VEHICLES, CELL CARRIERS OR COMBINATIONS THEREOF, IN THE FORM OF MICROSCAFFOLDS, AN INJECTABLE COMPOSITION CONTAINING SAID VEHICLES, AND ITS APPLICATIONS**
VERFAHREN ZUR HERSTELLUNG VON INJIZIERBAREN BIOKOMPATIBLEN WIRKSTOFFABGABE-VEHIKELN, ZELLTRÄGERN ODER KOMBINATIONEN DAVON, IN DER FORM VON MIKROGERÜSTEN, EINER INJEKTIONSFÄHIGEN ZUBEREITUNG DIESER VEHIKEL, SOWIE DESSEN ANWENDUNGEN
PROCÉDÉ D'OBTENTION DE VÉHICULES D'ADMINISTRATION DE MÉDICAMENTS BIOCOMPATIBLES INJECTABLES, DE VECTEURS CELLULAIRES OU DE COMBINAISONS DE CEUX-CI, SOUS LA FORME DE MICRO-ÉCHAFAUDAGES, COMPOSITION INJECTABLE CONTENANT LESDITS VÉHICULES, ET SES APPLICATIONS

(30) Priority: 22.02.2021 PL 43707821
(43) Date of publication of application: 27.12.2023
(73) Proprietor: Instytut Podstawowych Problemów Techniki Polskiej Akademii Nauk, 02-106 Warszawa (PL)
(72) Inventor: NAKIELSKI, Pawel, 02-237 Warszawa (PL); PAWLOWSKA, Sylwia, 80-333 Gdansk (PL); PRUCHNIEWSKI, Michal, 00-712 Warszawa (PL); URBANEK-SWIDERSKA, Olga, 05-092 Lomianki (PL); PIERINI, Filippo, 05-092 Lominaki (PL)
(74) Representative: Kondrat, Mariusz
(86) International application number: PCT/PL2022/050004
(87) International publication number: WO 2022/177454

(56) References cited:
- WO-A1-2017/089797
- KR-A- 20200 056 968
- KR-A- 20200 131 054
- US-A1- 2016 136 332
- BENJAMIN LI-PING LEE ET AL: "Femtosecond laser ablation enhances cell infiltration into three-dimensional electrospun scaffolds", ACTA BIOMATERIALIA, ELSEVIER, AMSTERDAM, NL, vol. 8, no. 7, 11 April 2012 (2012-04-11), pages 2648 - 2658, XP028511612, ISSN: 1742-7061, [retrieved on 20120419], DOI: 10.1016/J.ACTBIO.2012.04.023
- DANIELA STEFFENS ET AL: "Development of a biomaterial associated with mesenchymal stem cells and keratinocytes for use as a skin substitute", REGENERATIVE MEDICINE, vol. 10, no. 8, 1 November 2015 (2015-11-01), pages 975 - 987, XP055488021, ISSN: 1746-0751, DOI: 10.2217/rme.15.58
- PAWEL NAKIELSKI ET AL: "Laser-Assisted Fabrication of Injectable Nanofibrous Cell Carriers", SMALL, WILEY, HOBOKEN, USA, vol. 18, no. 2, 21 November 2021 (2021-11-21), pages n/a, XP072497947, ISSN: 1613-6810, DOI: 10.1002/SMLL.202104971

## Description

### TECHNICAL FIELD

The object of the invention is a method for obtaining injectable biocompatible drug delivery vehicles, cell carriers or combinations thereof, in the form of microscaffolds, an injectable composition containing said vehicles, and its applications.

### BACKGROUND ART

A key element in the process of tissue reconstruction or regeneration is the preparation of a suitable biomaterial for implantation. This biomaterial is a 3D scaffold enabling the growth of cells. It should be noted that the implanted scaffold can be precultured with patient cells and used as their carrier, or it can be populated with cells after implantation of the scaffold at the targeted site of injury. Polymers used as scaffolds should be biocompatible, biodegradable and biofunctional. Biocompatibility in the case of cellular scaffolds means the non-toxicity of their components (polymers, monomers, residual solvents and polymerisation initiators) and the physiological inertness of its biodegradation products. The components of such a system and its biodegradation products should not cause inflammation, other immunological reactions, changes in the composition of body fluids, or exhibit mutagenic or carcinogenic properties. Another vital criterion is biofunctionality, meaning the ability of the biomaterial to fulfil specific functions arising from its use. The overall porosity of the biomaterial and pore size are also significant. It is assumed that the size and arrangement of pores should ensure proper migration of tissue cells and transport of substances within the scaffold as well as deposition of new extracellular matrix. The morphological properties of scaffolds depend to a large extent on the method of their fabrication.

Many types of porous cellular scaffolds are described in the scientific literature, however not all of them possess the feature of injectability. The two main types of injectable cellular scaffolds are in situ crosslinked hydrogels and microparticles. In situ crosslinked hydrogels are the most common injectable scaffolds and can be classified based on the crosslinking method. Physically crosslinked hydrogels are less durable than chemically crosslinked hydrogels. The properties of hydrogel scaffolds in terms of regeneration largely depend on the chosen biomaterial, as it directs cell development, differentiation, and organisation in tissue engineering constructs. The biomaterial also provides physical support for cells and for the transport of chemical and biological signals required for the efficient formation of functional tissues.

Preclinical and clinical trials have provided a range of information on the interaction of cells with various commercially available biomaterials. Fibrin gels, Gelfoam^{™}, crosslinked (gelated) or liquid hyaluronate solution, hyaluronic acid sponges (e.g., HyStem^{®}, HYAFF), and Puramatrix^{®} have been studied. Hyaluronate in liquid or gel form has been found to demonstrate wider acceptance in clinical trials as a delivery vehicle of allogeneic mesenchymal stem cells or autologous stem cells derived from bone marrow. However, hydrogels as cell carriers for tissue regeneration have been chosen more often due to their easy handling and excellent safety profile, and not so often because of the desired biological effects of the hydrogel-cell construct or hydrogel degradation. Although hydrogels of natural origin offer numerous biological benefits, finding the right range of physical properties can be difficult. On the other hand, synthetic hydrogels crosslinked in situ may prove cytotoxic due to the use of toxic initiators, prepolymers, or monomers, and the increase in temperature during crosslinking. A common feature of hydrogels that may work against them is the semi-liquid form before the crosslinking step during injection, causing cell leakage from tissue, e.g., in intervertebral discs.

An injectable, biocompatible, and highly crosslinked hydrogel polymeric composition for treatment, characterised by shape memory after deformation by compression or dehydration is known from document EP2701745 B1**.** The composition has a porosity of at least 75%. The composition may be disc-shaped, cylindrical, square, rectangular or string-shaped. Preferably, it comprises an ingredient selected from the group consisting of hyaluronic acid, gelatin, heparin, dextran, locust bean gum, PEG, PEG derivative, collagen, chitosan, carboxymethylcellulose, pullulan, PVA, PHEMA, PNIPAAm or PAAc.

The literature indicates that apart from hydrogels, polymeric microparticles have gained attention as a new type of injectable scaffolds (Constantini M, Guzowski J, Zuk PJ, Mozetic P, De Panfilis S, Jaroszewicz J, Heljak M, Massimi M, Pierron M, Trombetta M, Dentini M, Swieszkowski W, Rainer A, Garstecki P, Barbetta A, Electric field assisted microfluidic platform for generation of tailorable porous microbeads as cell vehicles for tissue engineering. Advanced Functional Materials, 2018, https://doi.org/10.1002/adfm.201800874)*.*

Microspheres have been developed mainly for controlled drug delivery (e.g., Lupron Depot). Due to their small size and large specific surface area, microspheres can also act as injectable cell carriers in tissue engineering. Unlike hydrogels, microspheres allow cells to adhere and proliferate in vitro before the construct is injected into the damaged area, which is advantageous in specific applications. Microspheres can be formed from natural and synthetic polymers and inorganic materials. Like hydrogels, the physicochemical properties (e.g., hydrophilicity, strength and Young's modulus) and bioactivity (e.g., biocompatibility and degradation rate) of microspheres are governed by many factors such as composition, manufacturing process, particle size, surface morphology and microsphere modification methods. Besides their direct use as injectable cell carriers, microspheres can be used in tissue engineering together with hydrogels. A significant limitation in the use of microspheres is the lack of a fibrous three-dimensional surface morphology that would allow the cells to enhance their interaction with the material and function in tissue, e.g., in intervertebral discs. In addition, polymeric microspheres produce a higher amount of by-products during degradation than nanofibres, which may have long-term adverse effects on surrounding tissues.

One method of producing cellular scaffolds that has potential for clinical application is electrospinning. During electrospinning, a viscous stream of polymer solution is ejected from a cone-shaped droplet and then stretched in an electric field. Evaporation of the solvent causes the stretched jet to solidify and form nanofibres, which are then collected on a grounded collector in the form of nonwoven fabric. Electrospinning is a very popular method, as it offers the possibility of nanoscale material changes and is one of the few processes that are able to produce materials with high porosity. The method also offers the possibility to easy scaling up and industrial production.

To improve the surface properties of PLA or poly(lactide-co-glycolide) (PLGA), surface hydrolysis, surface coating, plasma treatment, chemical surface grafting, and other surface modification methods have been optimised over the past two decades. Of all of these methods, surface hydrolysis, especially surface alkaline hydrolysis using sodium hydroxide, has proven to be the most popular because it is simple, convenient, and effective.

Several examples of surface modification of polymers have been reported in the scientific literature. In a paper by Chen et al. (Chen F, Lee CN, Teoh SH. Nanofibrous modification on ultra-thin poly(e-caprolactone) membrane via electrospinning. Materials Science and Engineering: C, 2007, 27: 325-332), the authors describe a modification of the surface of polycaprolactone with 3 M of sodium hydroxide for about 3 hours, so that the contact angle decreased from 124° to 0°. Another paper (Campos DM, Gritsch K, Salles V, Attik GN, Grosgogeat B. Surface Entrapment of Fibronectin on Electrospun PLGA Scaffolds for Periodontal Tissue Engineering. Biores Open Access, 2014 1;3(3):117-126*)* describes the effect of concentration and incubation time in sodium hydroxide on the degree of fibronectin attachment to PLGA nanofibres. The authors showed that a concentration of 0.1 M and an incubation time of about 20 min was sufficient to functionalise the fibre surface with the protein.

The ability to modify and shape the surface of polymers and composite materials is of great importance for various biomedical applications. For example, the use of femtosecond laser ablation to create micropatterns on electrospun nanofibre materials can be successfully applied to the fabrication of complex polymeric biomedical devices, including scaffolds. In a paper by Paula et al. (Paula KT, Mercante LA, Schneider R, Correa DS., Mendonca CR. Micropatterning MoS2/Polyamide Electrospun Nanofibrous Membranes Using Femtosecond Laser Pulses. Photonics, 2019, 6(1): 3), laser ablation is described as an easy and convenient method for pattern formation (micropatterning) on electrospun polyamide (PA6) nanofibres that were modified with molybdenum disulfide (MoS₂). The effects of the laser pulse energy and structuring speed on the topography of the electrospun composite nanofibres were studied. The results demonstrated that micropores of a controlled size scale were formed using optimal laser parameters on electrospun nanofibres, while maintaining the initial morphology of nonwoven fabric. Most publications pertain to the formation of patterns on the surface of nonwoven fabrics (depressions) in which cells can be cultured (Lim YC, Johnson J, Fei Z, Wu Y, Farson D, Lannutti JJ, Choi HW, Lee LJ. Micropatterning and characterization of electrospun poly(ε-caprolactone)/gelatin nanofiber tissue scaffolds by femtosecond laser ablation for tissue engineering applications. Biotechnol. Bioeng., 108: 116-126. doi:10.1002/bit.22914*).*

Designing biomaterials that can mimic the extracellular matrix is crucial to enhancing therapeutic methods' efficacy. While covering superficial defects, e.g., skin with centimetre-sized nonwoven fabrics is relatively effective, filling hard-to-reach defects, including bone, cartilage, or intervertebral disc, has been found been unavailable. Microscaffolds of nanofibres, which can be inserted into defects by needle injection and which remain in the area of injection, are not available. Minimally invasive surgical methods are an alternative to classical or microsurgical operations, requiring general anaesthesia before surgery and gaining access to the target tissues. Thanks to the use of minimally invasive methods of treatment, the cause of pain is eliminated, as the small size of the skin incision helps in reducing the time spent in hospital to a minimum (often below 24 hours) and in returning to normal activity just a few days after the surgery. The technology of three-dimensional electrospun fibres, which can be injected minimally invasive, is of great added value, e.g., in cell therapies. In a paper by Michalek et al. (Michalek AJ, Buckley MR, Bonassar LJ, Cohen I, Iatridis JC. The effects of needle puncture injury on microscale shear strain. Spine J. 2010, 10(12): 1098-1105*),* it was shown that needle puncture of the fibrous ring of the intervertebral disc causes a drastic change in its deformation on a microscale. This change can directly initiate degeneration of the intervertebral disc due to an adverse change in mechanotransduction in the tissue. Therefore, it is necessary to introduce cellular scaffolds with devices/instruments that cause minimal tissue damage in many cases. To date, open surgery is the only way to implant conventional electrospun fibres, which have a fibrous structure similar to the extracellular matrix.

In this context, a method for producing a three-dimensional porous structure made of fragments made of the nonwoven fabric is known from document US20160136332A (EP3014005 B1). The method comprises cutting dry or wet nonwoven fabric made of nanofibres (e.g. electrospun nanofibres) into fragments using a laser (e.g. a pico- or femtosecond laser) and suspending the nonwoven fabric fragments in a liquid medium; or cutting nonwoven fabric made of nanofibres present in a liquid medium into fragments with a laser, which results in a suspension of nonwoven fabric fragments in the liquid medium; and at least partially removing the liquid medium; self-organisation leads to forming a three-dimensional porous structure of nonwoven fabric fragments. Preferably, prior to the laser treatment, the nonwoven fabric is treated with ethanol and/or UV light ensuring its sterilisation and modification of its physical and/or chemical properties and polymerisation. Preferably, the laser-cut nonwoven fabric is separated from the collector. Thus, during cutting, a vacuum table with the exhaust is used, and the cut fragments may escape with the drawn air and need to be captured. These aspects are not disclosed in document US20160136332A**.**

A method of manufacturing implants from electrospun nanofibres is known from document US20140294783**.** The method comprises the following steps of a) forming biodegradable, synthetic nanofibres; b) processing the nanofibres to produce nanofibre fragments; c) compressing the nanofibre fragments; and d) heating the nanofibre fragments to produce a porous, nanofibrous tissue scaffold. The cutting step involves fabrication of nanofibre fragments of random or homogeneous size. According to the authors of that document, such cutting may be performed manually or by milling, grinding, homogenisation and so forth. The authors do not mention the method of cutting by means of a laser beam, which is a crucial method for achieving microscale scaffolds.

Document KR20200056968 A discloses injectable nanofibre scaffolds and a method of fabricating them. According to the invention, electrospun nanofibres made of a biodegradable polymer are coated with a hydrogel precursor solution, and the whole is polymerised to produce nanofibres comprising a biopolymer core and a hydrogel coating. For electrospinning, a solution of a biodegradable polymer is placed in the inner nozzle, and a solution of polystyrene (PS) is placed in the outer nozzle. After electrospinning, fibres containing a core of biopolymer and a coating of PS are formed. The latter is then removed, leaving only the core of the biopolymer, which is then coated with hydrogel.

Microscaffolds containing a porous particle with a three-dimensional network of electrospun fibres containing a biocompatible polymer of cylindrical or polygonal prism shape with a diameter of 20-2000 µm and a height of 10-200 µm are known from document GB2544748A**.** Preferably, the porosity of the microscaffolds is > 75%. Preferably, the microscaffolds comprise ferromagnetic material (iron(II, III)oxide). Preferably, the microscaffolds may comprise a component selected from the group consisting of an extracellular matrix molecule (e.g. hyaluronidase, d-lysine, laminin, vitronectin, fibronectin, collagen), peptide, nucleic acid, fluorescent dye, streptavidin, biotin. The disclosed scaffolds may find applications in regenerative medicine, tissue engineering, screening for biological use or drug screening. A microscaffold may further comprise a magnetic material and a method of manipulating one or more such microscaffolds by exposing a composition comprising one or more such microscaffolds to a magnetic field of a magnet, thereby causing one or more microscaffolds in the composition to be attracted to the said magnet by magnetic attraction.

Similarly, the application WO2017089797 A1 discloses a microscaffold comprising a porous particle, wherein said particle comprises a three-dimensional network of fibres, which fibres comprise a polymer, and has a particle size less than or equal to 2000 µm. Said document further discloses a method of manipulating one or more microscaffolds, which method comprises exposing a composition which comprises one or more microscaffolds to a magnetic field of a magnet, and thereby causing the one or more microscaffolds in the composition to be attracted to said magnet by magnetic attraction.

### DISCLOSURE OF THE INVENTION

The problem of known injectable carriers is their mutual aggregation and sedimentation in solution, which may cause clogging of the needle during injection. Therefore, the aim of the present invention is to develop a new method of producing biocompatible mutually non-aggregating in vitro drug delivery vehicles and/or cell carriers for administration by injection.

The essence of the invention is a method of obtaining an injectable biocompatible drug delivery vehicle, cell carriers, or combinations thereof, in the form of microscaffolds, characterised in that it comprises the following steps:
a) preparation of a solution comprising a polymer and at least one solvent;
b) fibre formation with a fibre diameter between 50 nm and 10 µm on a flat collector in an electrospinning process;
c) laser cutting of the nonwoven fabric layer formed on the collector into individual detachable microscaffolds with a thickness between 0.01 and 0.2 mm and a cylindrical shape with a base diameter between 0.1 and 0.4 mm or a prism with a base edge length between 0.04 and 0.4 mm;
d) separation of microscaffolds from the collector;
e) chemical modification of the microscaffolds by suspending them in an aqueous NaOH solution of 0.001-1 M for 1-120 min;
f) rinsing excess NaOH from the microscaffold surface.

Preferably, the polymer is a biodegradable polymer selected from the group consisting of poly(L-lactide), poly(D-lactide), polycaprolactone, poly-(D,L)-lactide, poly-(L-lactide-co-D,L-lactide), polyglycolide, poly(lactide-co-glycolide), poly(lactide-co-caprolactone), polyurethane, chitosan, collagen, mixtures thereof or copolymers.

Preferably, in step b), fibres having a diameter of from 50 to 1000 nm are formed on the collector.

Preferably, in step b) a flat collector selected from the group consisting of a collector made of conductive material, a metal collector, a collector made of thin inorganic material, a glass collector, a collector covered with a layer of conductive material, a collector made of conductive material covered with a polymer film is used.

Preferably, in step a) active substance is added to the polymer solution in an amount of <1% by weight of the pure polymer.

Preferably, the added active substance is a growth factor selected from the group consisting of BMP-2, BMP-7, TGF, NGF or BDNF.

Preferably, in step c) the nanofibre layer is cut with a laser beam selected from the group consisting of an excimer laser, a picosecond laser or a femtosecond laser.

Preferably, in step c) the nanofibre layer is cut with a laser beam into prisms with a base being a figure selected from the group consisting of a triangle, square, rectangle, rhombus, parallelogram, trapezoid.

Preferably, the method according to the invention comprises step (g) which includes coating chemically modified, rinsed microscaffolds from step f) with a layer of a substance selected from the group consisting of chitosan, chondroitin sulfate, growth factor(s).

A further embodiment of the invention is an injectable delivery composition comprising a buffer and biocompatible in vitro mutually non-aggregating drug vehicles, cell carriers or combinations thereof, characterised in that the vehicles/carriers are electrospun fibre microscaffolds fabricated with the method according to the invention, wherein each microscaffold is a single vehicle/carrier with a thickness of 0.01 to 0.2 mm and the shape of a cylinder with a base diameter of 0.1 to 0.4 mm or a prism with a base edge length of 0.04 to 0.4 mm, the surface of which is modified chemically with NaOH solution, whereby the microscaffolds suspended in the buffer form a suspension of non-aggregating microparticles.

Preferably, the composition comprises mammalian cells placed on microscaffolds, whereby the said mammalian cells are selected from the group consisting of chondrocytes, osteoblasts, fibroblasts and stem cells.

Preferably, the buffer is selected from the group consisting of: buffer containing cell culture medium, phosphate buffer, HEPES buffer (2-[4-(2-hydroxyethyl)-1-piperazinyl]ethanesulfonic acid), MES buffer (2-morpholinoethanesulfonic acid), BIS-TRIS buffer ([bis(2-hydroxyethyl)imino]-tris(hydroxymethyl)methane).

A further embodiment of the invention is a composition comprising buffer and electrospun microscaffolds which, when suspended in the buffer, form a suspension of non-aggregating microparticles, and each microscaffold is a single vehicle/carrier with a thickness of 0.01 to 0.2 mm and the shape of a cylinder with a base diameter of 0.1 to 0.4 mm or a prism with a base edge length of 0.04 to 0.4 mm, the surface of which is modified chemically with NaOH solution, for use in the treatment of bone, cartilage or intervertebral disc injuries.

Unexpectedly, it was found that the parameters of the microscaffolds according to the invention, i.e. the thickness of the microscaffolds, the diameter of the base of the cylindrical microscaffolds and the length of the edge of the base of the prism-shaped microscaffolds must meet certain conditions.

Lower values of disclosed range of thickness, diameter and edge length of the base of the microscaffolds according to the invention result from physical limitations of the material, e.g. its strength, which translates into the disintegration of such small micro-objects under the influence of even slight shearing stress encountered during mixing with the use of a stirrer. The smallest microscaffolds that do not disintegrate in liquid and after chemical treatment in the shape of a cylinder have a base diameter of 0.1 mm, while the minimum edge length for cubes, triangles or hexagons is 0.04 mm. The benefit of such a small size (0.04 mm for a prism) is that polymeric microscaffolds containing, for example, drugs can be injected through 30G size needles, i.e. needles with an internal diameter of 0.159 mm, without them clogging the needles. When injecting a microscaffold-cell construct, the microscaffolds should be sufficiently larger to provide surface area for cell growth. The optimal size of microscaffolds that can be administered with a 26G size needle is 0.14 mm. As far as cell therapies are concerned, which are usually introduced into the body via a needle, the use of a smaller size needle causes less tissue trauma (e.g. when puncturing the fibrous ring of the intervertebral disc) and minimises cell leakage through the puncture created by the needle insertion. Only the use of a small needle with a size of 20-30G can ensure that the procedure of delivery of a microscaffold-cell construct is administered in a minimally invasive manner, reducing side effects of cell therapy and thus reducing the length of the patient's hospitalisation.

The invention provides the following benefits:
- The method according to the invention serves to fabricate microscaffolds of predetermined shapes and sizes, which can be suspended in water in which they float freely and evenly throughout its volume without aggregating;
- Hydrolysis within the method according to the invention protect fabricated vehicles/carriers from agglomeration and deposition on the walls, so it is possible to inject aqueous suspensions of microscaffolds through a needle with a small size of 20-30G without clogging during injection;
- Fabricated microscaffolds provide biocompatible platforms that act as scaffolds for cells while allowing the passage of nutrients, oxygen and metabolites;
- Fabricated microscaffolds can be used as cellular scaffolds populated in vitro with cells taken from patients to replace small bone defects or to regenerate the intervertebral disc in the same patients (in this case, patients can be cell donors and recipients);
- Fabricated microscaffolds can be used as scaffolds for cells in bioreactors for expansion of cell cultures;
- Fabricated microscaffolds for injection allow the drug to be released directly at the application site and the dose is reduced, which limits systemic toxicity;
- Microscaffolds fabricated with the method according to the invention show good manageability, cohesiveness and bioactivity;
- No cytotoxicity due to the use of femtosecond laser cutting which enables cold ablation;
- The composition according to the invention containing microscaffolds obtained with the method according to the invention can be used as biological tissue filler for the treatment of bone, cartilage and intervertebral disc injuries.

### BRIEF DECRIPTION OF DRAWINGS

The invention is illustrated in examples and in figures, whereby **Fig. 1** shows microscaffolds obtained with the method according to the invention (SEM analysis, magnification x85, scale 200 µm); **Fig. 2** shows the morphology of nonwoven fabric from microscaffolds before and after surface modification with the method according to the invention (SEM analysis, magnification x5000, scale 5 µm); **Fig. 3** shows solutions of hyaluronic acid with different concentrations of microscaffolds having a 0.14 mm by 0.14 mm quadrilateral as the base, 1 minute after mixing the solution; the absence of microscaffolds at the bottom of the sample with unmodified microscaffolds and accumulation at the phase boundary is visible; **Fig. 4** shows the results of injectability tests of modified microscaffolds according to the invention, having a 0.14 mm by 0.14 mm quadrilateral as the base, through needles of size 23, 24 and 26G; **Fig. 5** shows the profile of release of a model substance from microscaffolds made of PLGA, PLLA and PLCL polymers; **Fig. 6** shows a viability analysis of L929 mouse fibroblasts from in vitro cultures with microscaffolds fabricated with the method according to the invention (50 µm scale), where live L929 fibroblasts are visible on the surface of the said microscaffolds; **Fig. 7** shows the adhesion of the L929 mouse fibroblast line to microscaffolds according to the invention (SEM analysis, magnification x 500, scale 50 µm), where A) denotes unmodified control scaffolds, B) denotes modified microscaffolds fabricated with the method according to the invention.

### DESCRIPTION OF EMBODIMENTS

### EXAMPLES

### Example 1.

In this example, 9 parts by weight (PBW) of poly(L-lactide-co-caprolactone) (PLCL, 70% L-lactide, 30% caprolactone) was dissolved in 85.5 PBW of chloroform, and then 5.2 parts of dimethylformamide was added. The polymer was dissolved at room temperature until completely dissolved (for a minimum of 24 hours).

Although in this preferred example PLCL was chosen for fabrication of scaffolds, other polymers (e.g., poly(L-lactide), poly(D-lactide), polycaprolactone, poly-(D,L)-lactide, poly-(L-lactide-co-D,L-lactide), polyglycolide, poly-(lactide-co-glycolide), polyurethane, chitosan, collagen, mixtures thereof or copolymers) may also be used in the method according to the invention.

The solution thus obtained was used to produce nonwoven fabric. However, in the method according to the invention, the said nonwoven fabric is obtained by electrostatic spinning (electrospinning), in which streams of polymer solution are stretched in an electric field to a dimension of fractions of a micrometre.

In this example, the material was spun at a voltage of 15 kV and a distance of 20 cm from the grounded flat glass collector. In the method according to the invention, other flat collectors can also be used (e.g., a metal collector, a collector covered with a metal layer or a collector made of conductive material covered with a polymer film).

The pump volume flow rate was 0.8 ml/h. The materials were prepared at room temperature (approx. 22-24°C). Nanofiber material with a thickness of 0.1 mm and a porosity of about 80% was obtained. In the next step, the material together with the collector was placed on the laser processing table. In this example, a femtosecond laser was used, but other lasers (e.g., picosecond laser or excimer laser) can also be used.

A material cutting pattern was introduced into the laser software, which in this example included a series of perpendicular cuts (in this example, the line offset in the resulting lattice was 0.04 mm in the vertical and horizontal direction). After setting the laser power, the number of beam runs, and the cutting speed, the result of the laser processing was a prism-shaped microscaffold with a 0.04 mm by 0.04 mm quadrilateral at its base. By changing the laser settings, it is possible to obtain a different shape of microscaffolds (e.g., it is possible to obtain cylinder-shaped microscaffolds or prism-shaped microscaffolds with a quadrilateral, triangle, trapezoid, rhombus, parallelogram, square or other rectangle or pentagon, hexagon, octagon or other polygons as the base). Thus, scaffolds of different sizes and shapes can be obtained with the method according to the invention. Depending on the selected shape, the parameters of the microscaffolds according to the invention (i.e. the thickness of microscaffolds, diameter of the base of cylindrical microscaffolds, and length of the edge of the base of prism-shaped microscaffolds), the microscaffolds must meet certain conditions resulting from physical limitations of deployed materials (i.e., biodegradable polymers). This means that the smallest chemically-treated cylinder microscaffolds not degrading in a liquid have a base with a diameter of 0.1 mm, and prism microscaffolds have a base with a minimum length edge of 0.04 mm. Minimum dimensions to ensure the stability of scaffolds were determined experimentally.

Cutting of nonwoven fabric deposited on the collector in the laser cutting process according to the invention takes place in ventilated chambers. Moreover, cutting of nonwoven fabric bound to the collector used in the method has an advantage over cutting nonwoven fabric alone. Before laser processing, materials are fixed to the table with the use of a vacuum, while in the chamber itself, a fume extractor is used. When cutting nonwoven fabric without a collector, microscaffolds are released from nonwoven fabric and sucked into the fume extractor, which significantly reduces the number of obtained microscaffolds.

Polymers used to manufacture nanofibres are hydrophobic materials; thus, the water contact angle on nonwoven fabric is higher than 90° and often exceeds 120°. Therefore, material cut on the collector is chemically modified by suspending microscaffolds in aqueous sodium hydroxide solution over a specified time and then rinsing with deionised (or distilled) water to remove the sodium hydroxide. Modification is carried out in Eppendorf or Falcon tubes or in glass containers by mixing in a laboratory shaker, which reduces the time of the modification process. During the chemical treatment, a gradual modification of microscaffolds is observed, and their sedimentation from the gas-liquid interface and their accumulation on the bottom of the tube. When the absence of microscaffolds on the surface between phases is observed, the microscaffolds are centrifuged, and the fluid with hydroxide (modifying substance) is removed. Too long incubation of microscaffolds in hydroxide solution leads to rapid degradation of the polymer, while in the case of too high concentrations of, e.g., sodium hydroxide, immediate dissolution of microscaffolds is observed.

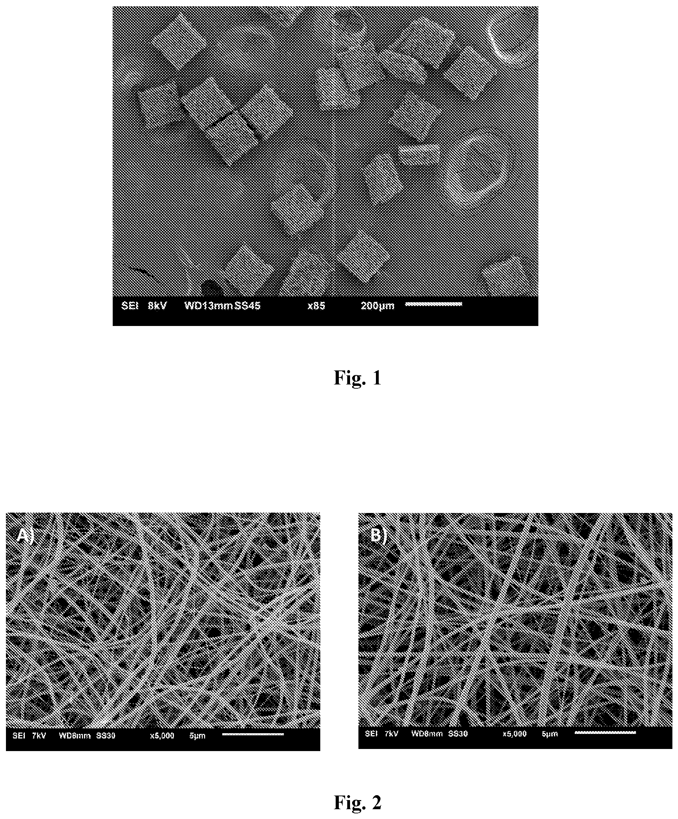

Therefore, in this example, after cutting the nonwoven fabric into microscaffolds of predefined shapes, the glass collector with the cut material was placed in a vessel (Falcon tube 50 ml) with an aqueous solution of sodium hydroxide (NaOH) of 0.5 M and the microscaffolds were removed from the collector surface and then modified, which lasts about 25 min. The microscaffolds were then centrifuged for 5 min at 13500 rpm, and the sodium hydroxide solution was removed. Next, the modified microscaffolds were suspended in deionised water to rinse the sodium hydroxide, assess whether the microscaffolds accumulated on the water-air surface and, when all microscaffolds were modified, the procedure of centrifugation and water rinsing was repeated until a neutral pH level was obtained. The microscaffolds (before and after treatment) were subjected to quality control using an electron microscope. Fig. 1 shows the scaffolds after chemical modification. A comparison of the fibre morphology of the microscaffolds before and after chemical treatment with sodium hydroxide is shown in Fig. 2.

Thanks to the described method, microscaffolds of predefined shape and size can be suspended in water (or in complex buffer), in which they float freely and evenly throughout their volume. Thus, it is possible to inject aqueous suspensions of microscaffolds with needles of small sizes of 20-30G, in particular 24-27G, without clogging the needle during injection. The amount of obtained microscaffolds can be counted using counting chambers (e.g., Nageotte chambers), which enables the selection of an appropriate concentration of microscaffolds for injection.

In this example, NaOH-modified prism-shaped microscaffolds with a height of 0.1 mm (corresponding to the thickness of the microscaffold) and a quadrilateral base of 0.04 mm x 0.04 mm were obtained and suspended in water.

### Example 2.

The method is similar to the one in example 1. A material cutting pattern was introduced into the laser software, which in this example included a series of perpendicular cuts (the line offset in the resulting lattice was 0.4 mm in the vertical and horizontal directions). After setting the laser power, the number of beam runs, and the cutting speed, the result of the laser processing was a prism-shaped microscaffold with a 0.4 mm by 0.4 mm quadrilateral at its base. Chemical modification of the cut scaffolds was carried out in an aqueous NaOH solution of 0.001 M concentration for 120 min.

### Example 3.

In this example, 6.25 PBW of poly(L-lactide) (PLLA) was dissolved in 84.38 PBW of chloroform, and then 9.37 parts of dimethylformamide was added. Other polymers (e.g., polyglycolide, poly(D-lactide), poly(D,L)-lactide, chitosan, collagen) can be used in the method according to the invention. The polymer was dissolved at room temperature for at least 24 hours. The solution thus obtained was used for electrospinning. The material was spun at a voltage of 17 kV and a distance of 15 cm from the grounded flat glass collector. However, other types of flat collectors can be used, e.g., a metal collector or collector covered with a metal layer. The pump volume flow rate was 0.8 ml/h. The materials were prepared at room temperature (approx. 22-24°C). Nanofibrous material with a thickness of 0.09 mm and a porosity of about 85% was obtained. In the next step, the material together with the collector was placed on the laser processing table. A cutting scheme of the material was introduced into the laser software. It included a series of perpendicular cuts (the offset of the lines in the resulting lattice was 0.14 mm in the vertical and horizontal directions). After setting the laser power, the number of beam runs, and the cutting speed, the result of the laser processing was a quadrilateral 0.14 mm by 0.14 mm microscaffold. Next, the glass collector and cut material were placed in a vessel with deionised water, and the microscaffolds were removed from the collector surface. The microscaffolds were left in water for 1 hour so that water molecules could reach deeper layers of the nanofibres forming the microscaffolds, which leads to the improvement of microscaffold penetration through an aqueous solution. After 1 hour, the microscaffolds were separated by centrifugation (20 minutes, 13500 rpm). If this step fails, liquid can be removed with a very thin needle (e.g., 31G) without removing the microscaffolds. After removing the water, the microscaffolds accumulated at the bottom of the Eppendorf tube were suspended in 0.1 M sodium hydroxide solution. The sample thus prepared was placed in a shaker to intensify the modification process, whereby its progress was checked every few minutes. The chemical modification was considered complete when the sedimentation of microscaffolds at the bottom of the Eppendorf tube from the gas-liquid boundary surface was observed. The average time for modification of PLLA fibre microscaffolds in 0.1 M NaOH solution was 45 min. Then, the aqueous NaOH solution together with the dispersed microscaffolds was centrifuged (2 min, 13500 rpm) to remove the sodium hydroxide solution. The microscaffolds were then suspended in water to rinse the sodium hydroxide, and the centrifugation and rinsing procedure was repeated until a neutral pH level was achieved (involving an average of 6 rinsing cycles). The microscaffolds (before and after treatment) were subjected to quality control using an electron microscope.

### Example 4.

In this example, 5 PBW of polycaprolactone (PCL) was dissolved in 95 PBW of hexafluoroisopropanol (HFIP). The polymer was dissolved at room temperature for at least 24 hours. The solution thus obtained was used for electrospinning. The material was spun at 12 kV and a distance of 15 cm from the grounded flat glass collector (other options: metal collector). The pump volume flow rate was 0.8 ml/h. The materials were prepared at room temperature (approx. 22-24°C). Nanofibre material with a thickness of 0.05 mm and a porosity of about 80% was obtained. In the next step, the material together with the collector was placed on the femtosecond laser processing table. A cutting scheme of the material was introduced into the laser software. It included a series of perpendicular cuts (the offset of the lines in the resulting lattice was 0.05 mm in the vertical and horizontal directions). After setting the laser power, the number of beam runs, and the cutting speed, the result of the laser processing was a quadrilateral 0.05 mm by 0.05 mm microscaffold. Next, the glass collector and cut material were placed in a vessel with deionised water, and the microscaffolds were removed from the collector surface. The microscaffolds were left in water for 1 hour so that water molecules could reach deeper layers of the nanofibres forming the microscaffolds, which leads to the improvement of microscaffold penetration through an aqueous solution. After 1 hour, the microscaffolds were separated by centrifugation (20 minutes, 13500 rpm) (or, in case of failure, the liquid was removed with a very thin needle (e.g., 31G) without removing the microscaffolds). After removing the water, the microscaffolds accumulated at the bottom of the Eppendorf tube were suspended in a 1 M sodium hydroxide solution. The sample thus prepared was placed in a shaker to intensify the modification process, whereby its progress was checked every few minutes. The chemical modification was considered complete when the sedimentation of microscaffolds at the bottom of the Eppendorf tube from the gas-liquid boundary surface was observed. The average time for modification of PCL fibre microscaffolds in 1 M NaOH solution was 40 min. Then, the aqueous NaOH solution together with the dispersed microscaffolds were centrifuged (2 min, 13500 rpm) to remove the sodium hydroxide solution. The microscaffolds were then suspended in water to rinse the sodium hydroxide, and the centrifugation and rinsing procedure was repeated until a neutral pH level was achieved (6 rinsing cycles).

The microscaffolds (before and after treatment) were subjected to quality control using an electron microscope.

### Example 5.

In this example, 4 PBW of poly(lactide-co-glycolide) (PLGA) was dissolved in 96 PBW of hexafluoroisopropanol (HFIP). The polymer was dissolved at room temperature for at least 24 hours. The solution thus obtained was used for electrospinning. The material was spun at 15 kV and a distance of 14 cm from the grounded flat glass collector (other options: metal collector). The pump volume flow rate was 0.8 ml/h. The materials were prepared at room temperature (approx. 22-24°C). Nanofibre material with a thickness of 0.1 mm and a porosity of about 76% was obtained. In the next step, the material together with the collector was placed on the laser processing table. A cutting scheme of the material was introduced into the laser software. It included a series of perpendicular cuts (the offset of the lines in the resulting lattice was 0.14 mm in the vertical and horizontal directions). After setting the laser power, the number of beam runs, and the cutting speed, the result of the laser processing was a quadrilateral 0.14 mm by 0.14 mm microscaffold. Next, the glass collector and cut material were placed in a vessel with deionised water, and the microscaffolds were removed from the collector surface. The microscaffolds were left in water for 1 hour so that water molecules could reach deeper layers of the nanofibres forming the microscaffolds, which leads to the improvement of microscaffold penetration through the aqueous solution. After 1 hour, the microscaffolds were separated by centrifugation (20 minutes, 13500 rpm) (or, in case of failure, the liquid was removed with a very thin needle (31G) without removing the microscaffolds). After removing the water, the microscaffolds accumulated at the bottom of the Eppendorf tube were suspended in 0.05 M sodium hydroxide solution. The sample thus prepared was placed on a shaker to intensify the modification process, checking its progress every few minutes. The chemical modification was considered complete when the sedimentation of microscaffolds at the bottom of the Eppendorf tube from the gas-liquid boundary surface was observed. The average time for modification of PLGA fibre microscaffolds in 0.05 M NaOH solution was 40 min. Then, the aqueous NaOH solution together with the dispersed microscaffolds was centrifuged (2 min, 13500 rpm) to remove the sodium hydroxide solution. The microscaffolds were then suspended in water to rinse the sodium hydroxide, and the centrifugation and rinsing procedure was repeated until a neutral pH level was achieved (6 rinsing cycles).

The microscaffolds (before and after treatment) were subjected to quality control using an electron microscope.

### Example 6.

In this example, 0.22 PBW of collagen was dissolved in 75 PBW of ethanol and 24.78 PBW of water, also containing 30 mM hydrochloric acid. The polymer was dissolved at room temperature for at least 24 hours. The solution thus obtained was used for electrospinning. The material was spun at 12 kV and a distance of 10 cm from the grounded flat metal collector coated with a polymer film. The volume output of the pump was 0.3 mL/h. The materials were prepared at room temperature (approx. 22-24°C) and 10% relative humidity. Nanofibre material with a thickness of 0.2 mm was obtained.

In the next step, the material together with the collector was placed on an excimer laser processing table. A cutting scheme of the material was introduced into the laser software. It provided cylinders with a base diameter of 0.4 mm.

Then, the collector and cut material were placed for 20 min in a desiccator containing a vessel with 4 mL of ammonia water, and in the next step, it was placed in a vessel with deionised water, and the microscaffolds were removed from the surface of the collector. The microscaffolds were left in water for 1 hour so that water molecules could reach deeper layers of the nanofibres forming the microscaffolds, which leads to the improvement of microscaffold penetration through the aqueous solution. After 1 hour, the microscaffolds were separated by centrifugation (20 minutes, 13500 rpm). After removing the water, the microscaffolds accumulated at the bottom of the Eppendorf tube were suspended in 0.01 M sodium hydroxide solution, and a chemical modification that lasted 1 min was conducted.

Then, the aqueous NaOH solution and the dispersed microscaffolds were centrifuged (2 min, 13500 rpm) to remove the sodium hydroxide solution. The microscaffolds were then suspended in water to rinse the sodium hydroxide, and the centrifugation and rinsing procedure was repeated until a neutral pH level was achieved (6 rinsing cycles). The microscaffolds (before and after treatment) were subjected to quality control using an electron microscope.

### Example 7.

In this example, 8 PBW of chitosan was dissolved in 83 PBW of acetic acid and 9 PBW of water. The polymer was dissolved at 30°C for a minimum of 24 hours. The solution thus obtained was used for electrospinning. The material was spun at a voltage of 25 kV and a distance of 7 cm from the grounded flat glass collector. The volume output of the pump was 1 mL/h. The materials were prepared at room temperature (approx. 22-24°C). Nanofibre material with a thickness of 0.05 mm was obtained.

A cutting scheme of the material was introduced into the laser software. It provided prisms with a triangle of 0.2 mm edge length as the base. The collector and cut material were placed in a vessel with deionised water, and the microscaffolds were removed from the collector surface. The microscaffolds were left in water for 1 hour so that water molecules could reach deeper layers of the nanofibres forming the microscaffolds, which leads to the improvement of microscaffold penetration through the aqueous solution. After 1 hour, the microscaffolds were separated by centrifugation (20 minutes, 13500 rpm). After removing the water, the microscaffolds accumulated at the bottom of the Eppendorf tube were suspended in 0.01 M sodium hydroxide solution, and a chemical modification that lasted 60 min was conducted. Then, the aqueous NaOH solution, along with the dispersed microscaffolds were centrifuged (2 min, 13500 rpm) in order to remove the sodium hydroxide solution. The microscaffolds were then suspended in water to rinse the sodium hydroxide, and the centrifugation and rinsing procedure was repeated until a neutral pH level was achieved (6 rinsing cycles). The microscaffolds (before and after treatment) were subjected to quality control using an electron microscope.

### Example 8.

In this example, 7 PBW of PLCL was dissolved in 93 PBW of hexafluoroisopropanol (HFIP) and left on a magnetic stirrer for 24 h until the polymer was completely dissolved. The solution thus obtained was used for electrospinning. The material was spun at a voltage of 15 kV and a distance of 15 cm from the grounded flat glass collector. The volumetric output of the pump was 0.8 mL/h. The material was electrospun at ambient temperature (approx. 22-24°C). The obtained fibres were homogeneous along their entire length, with an average size of 700 nm. Finally, the material on the glass collector was placed on a laser processing table and subjected to femtosecond laser cutting into microscaffolds with the shape and dimensions set in the software (e.g., a quadrilateral with a size of 0.14 mm by 0.14 mm). Next, the glass collector and cut material were placed in a vessel with deionised water, and the microscaffolds were removed from the collector surface and put into the water. First, the microscaffolds were left in the water for 1 hour to be wetted. After that, they were centrifuged (20 minutes, 13500 rpm) or, if unsuccessful, the liquid was removed with a very fine needle (31G) without removing the microscaffolds. The microscaffolds were then covered with 0.05 M NaOH solution for 5 min in order to hydrolyse the surface. After 5 min, the NaOH solution was centrifuged, and the microscaffolds were rinsed with water to remove residual NaOH. Rinsing was repeated 3 times, each lasting about 15 minutes, until the pH of the solution was neutral. The thus prepared scaffolds were placed in NHS/EDC solution (50 mM NHS and 50 mM EDC) and left for 1 hour. After that, the NHS/EDC solution was centrifuged, and the microscaffolds were rinsed in deionised water for 5 minutes. Finally, the microscaffolds were placed in a previously prepared growth factor solution. In this example, the growth factor was transforming growth factor beta 1 (TGF-β1) with a concentration of 10 ng/ml in 0.01 M phosphate buffer. The microscaffolds were incubated in the growth factor solution at the temperature of 4°C for 24 hours. After this time, the microscaffolds were centrifuged again and rinsed in deionised water. Finally, the microscaffolds were dried and stored at 4°C.

### Example 9.

The method is similar to the one in example 8, except that 5 PBW of PLCL was dissolved in 95 PBW of HFIP and left on a magnetic stirrer for 24 hours until the polymer was completely dissolved. The resulting solution was used for electrospinning, where the material was spun at 15 kV and a distance of 15 cm from the grounded flat glass collector. The volume output of the pump was 0.5 mL/h. The materials were electrospun at ambient temperature (approx. 22-24°C). The obtained fibres were homogeneous along their entire length and had a diameter of 100 nm. The method, according to the invention, also obtains fibres with a smaller diameter, e.g., 50 nm.

### Example 10.

The method is similar to the one in example 8, except that 15 PBW of PLCL was dissolved in 85 ml of 2,2,2-trifluoroethanol and left on the magnetic stirrer for 24 hours until the polymer was completely dissolved. The resulting solution was used for electrospinning, where the material was spun at 17 kV and a distance of 12 cm from the grounded flat glass collector. The pump volume flow rate was 0.8 ml/h. The materials were electrospun at ambient temperature (approx. 22-24°C). The obtained fibres were homogeneous along their entire length and had a diameter of 10 µm.

### Example 11.

In order to prepare a solution containing 95% w/w PLGA and 5% w/w chitosan, a two-step method was used to dissolve the components in hexafluoroisopropanol (HFIP) to finally achieve a solution with a total polymer concentration of 4% w/w relative to the solvent. First, chitosan was dissolved in a water bath at 50°C for about 24 hours. Then, PLGA was added to the chitosan solution, which was cooled to room temperature and left to dissolve for another 24 hours. The solution thus obtained was used for electrospinning. The material was spun at 17 kV and a distance of 15 cm from the grounded flat glass collector (other options: metal collector). The volume output of the pump was 1.5 mL/h. The materials were prepared at room temperature (approx. 22-24°C). Depending on the voltage and feed rate of the solution, fibres could achieve an average size of 350 nm. Finally, the material on the glass collector was placed on a laser processing table and subjected to laser cutting into microscaffolds with the shape and dimensions set in the software (e.g., a quadrilateral with a size of 0.14 mm by 0.14 mm). Next, the glass collector and cut material were placed in a vessel with deionised water, and the microscaffolds were removed from the collector surface and placed in the water. The microscaffolds were left in the water for 1 hour to be wetted. After that, they were centrifuged (20 minutes, 13500 rpm) in order to be separated (2 min, 13500 rpm), and then covered with 0.05 M NaOH solution and left for 5 min (surface hydrolysis). The centrifugation procedure was then repeated, and the microscaffolds were covered with water to rinse the NaOH. Rinsing was repeated 3 times, each time for about 15 minutes. The thus prepared scaffolds were placed in NHS/EDC solution (50 mM NHS and 50 mM EDC) and left for 1 hour. After that, the NHS/EDC solution was centrifuged, and the microscaffolds were rinsed in water for 5 minutes. In this example, the microscaffolds were finally placed in a previously prepared 1 mg/ml chondroitin sulfate solution with a concentration of 1 mg/ml in 0.02 M NaCl solution for 24 hours. However, other solutions can be used instead of chondroitin sulphate, e.g., chitosan or growth factor solutions. After that, the microscaffolds were centrifuged again and rinsed three times (once in 0.02 M NaCl solution and twice in deionised water). Finally, the microscaffolds were dried and stored at 4°C.

### Example 12.

### Fabrication of microscaffolds with active substance.

In this example, 9 PBW of poly(L-lactide-co-caprolactone) (PLCL, 70% L-lactide, 30% caprolactone) was dissolved in 85.5 PBW of chloroform and then 5.2 PBW of dimethylformamide was added. The polymer was dissolved at room temperature for at least 24 hours. 5 mg of SPAN-80 surfactant was added to 1 gram of polymer solution and stirred. Then, an active ingredient, which was a growth factor in 0.01 M phosphate buffer, was added to the polymer-surfactant solution in two portions of 25 µL each. In this preferred example, the growth factor used was bone morphogenetic protein 2 (BMP2); however other active substances, including other growth factors (e.g., bone morphogenetic protein 7 BMP-7, nerve growth factor NGF or brain-derived neurotrophic factor BDNF), can also be used. In the method according to the invention, different concentrations of the active substance can be used, but preferably the amount of active substance should be <1% relative to the weight of the pure polymer used for electrospinning.

The whole material was mixed and shaken until a homogeneous and stable emulsion of water in the organic phase was formed. The material was spun at a voltage of 15 kV and a distance of 20 cm from the grounded flat glass collector. The volume output of the pump was 0.8 mL/h. The materials were prepared at room temperature (approx. 22-24°C). Nanofibre material with a thickness of 0.1 mm and a porosity of about 80% was obtained. In the next step, the material together with the collector was placed on the laser processing table. A cutting scheme of the material was introduced into the laser software. It included a series of perpendicular cuts (the offset of the lines in the resulting lattice was 0.05 mm in the vertical and horizontal directions). After setting the laser power, the number of beam runs, and the cutting speed, the result of the laser processing was a quadrilateral 0.05 mm by 0.05 mm microscaffold. Further, the glass collector and cut material were placed in a vessel (Falcon tube 50 ml) with an aqueous solution of sodium hydroxide of 0.1 M, and the microscaffolds were removed from the collector surface and then modified whereby the modification lasted for about 5 min. The microscaffolds were then centrifuged for 5 min at 13500 rpm, and the sodium hydroxide solution was removed. The microscaffolds were then suspended in water to rinse the sodium hydroxide and to assess whether the microscaffolds accumulated on the water surface at the border. If all microscaffolds were modified, centrifugation and rinsing were repeated until a neutral pH level was obtained. The microscaffolds (before and after treatment) were subjected to quality control using an electron microscope.

### Example 13.

The composition according to the invention contains microscaffolds obtained with the method according to the invention and buffer, whereby the microscaffolds were sterilised with 85% ethanol. After centrifuging and removing the ethanol, the microscaffolds were irradiated with UV light (2 x 30 min) and then suspended in a sterile buffer.

In this preferred example, the composition comprises chemically modified (with 1 M NaOH solution, for 2 min) microscaffolds of polycaprolactone (PCL) having a porosity of about 80% and a prism shape with a height of 0.1 mm (corresponding to the thickness of the microscaffold) and a quadrilateral base of 0.14 mm x 0.14 mm, which were suspended in 0.5% hyaluronic acid. For the suspension of the composition according to the invention, other buffers understood to be standard laboratory buffers, solutions, in particular aqueous solutions, and water may be used, e.g., buffer containing culture medium for cell cultures, phosphate buffer, HEPES buffer (2-[4-(2-hydroxyethyl)-1-piperazinyl]ethanesulfonic acid), MES buffer (2-morpholinoethanesulfonic acid), BIS-TRIS buffer ([bis(2-hydroxyethyl)imino]-tris(hydroxymethyl)methane), poloxamer, collagen gel or alginate gel.

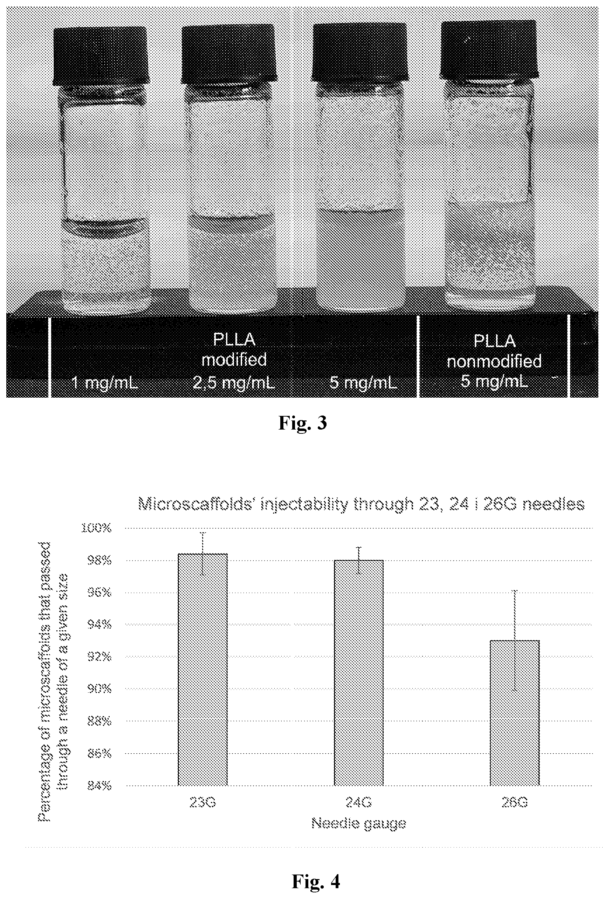

The said microscaffolds float freely and homogeneously in the volume of the buffer (i.e. 0.5% hyaluronic acid solution) without accumulating at the solution-air border and without aggregating (Fig. 3). Fig. 3 shows solutions of hyaluronic acid with different concentrations of microscaffolds having a 0.14 mm by 0.14 mm quadrilateral as the base, 1 minute after mixing the solution; the absence of microscaffolds at the bottom of the sample with unmodified microscaffolds and accumulation at the phase boundary is visible.

Thus, it is possible to inject aqueous suspensions of microscaffolds with needles of small sizes (withing the range of 23-26G) without clogging the needle during injection. The results of injectability tests of modified microscaffolds according to the invention, having a 0.14 mm by 0.14 mm quadrilateral as the base, through needles of sizes 23, 24, and 26G, are presented in Fig. 4. As indicated in Fig. 4, satisfactory injectability (i.e., above 90%) of microscaffolds according to the invention was achieved for all tested needle types.

Regarding the determination of the desired concentration of microscaffolds in the buffer, they can be counted using a Nageotte chamber (or other counting chambers) to determine the appropriate concentration of carrier/vehicle for injection or inoculation of cell cultures. In this example, the composition contains 600 microscaffold/mL of hyaluronic acid solution.

### Example 14.

In this example of the invention, the composition, according to the invention, comprises microscaffolds fabricated with a method according to the invention with mammalian cells placed thereon and buffer containing cell culture medium.

In this preferred example, the said mammalian cells are human bone marrow stem cells. The composition, according to the invention, may comprise microscaffolds on which other cell types (e.g., chondrocytes, osteoblasts, or fibroblasts) are also deposited.

In this example of the invention, microscaffolds obtained with the method according to example 6 were used. They were sterilised with 85% ethanol before cell sedimentation. After centrifuging and removing the ethanol, the microscaffolds were irradiated with UV light (2 x 30 min) and then suspended in a sterile buffer.

In order to deposit stem cells on the microscaffolds according to the invention, bone marrow stem cells in an amount of 15-103/mL were cultured in non-adherent wells of a culture plate together with microscaffolds fabricated according to example 6. The cultivation was performed in DMEM medium (Dulbecco's Modified Eagle Medium) supplemented with 10% bovine serum at a temperature of 37°C in an atmosphere containing 95% air and 5% CO2. After a 24 h incubation, the culture was harvested and centrifuged. Centrifuged microscaffolds were then washed to rinse non-deposited cells. The microscaffolds were then resuspended in buffer, which in this example is EMEM (Eagle's Minimum Essential Medium) cell culture medium supplemented with 10% bovine serum. The said microscaffolds float freely and homogeneously in the volume of the buffer and do not aggregate.

Although in this preferred example, EMEM medium supplemented with 10% serum was used as a buffer, the said buffer can also be another medium (alone or with additives including, e.g., bovine serum, antibiotics, vitamins, etc.) typically used for mammalian cell cultures. Whereby, selection of the medium depends on the mammalian cells to be deposited on the microscaffolds according to the invention.

### Example 15.

The composition according to the invention contains microscaffolds obtained with the method according to the invention and buffer, whereby the microscaffolds were sterilised with 85% ethanol. After centrifuging and removing the ethanol, the microscaffolds were irradiated with UV light (2 x 30 min) and then suspended in a sterile buffer. In this preferred example, the composition comprises chemically modified microscaffolds (chemical modification with 0.5 M NaOH solution in 5 min.) made of poly(L-L-lactide-co-caprolactone) (PLCL) with incorporated an active substance, which is Rhodamine B in the amount of 1% relative to the weight of the polymer. The shape of the microscaffolds was a prism of 0.8 mm height (corresponding to the thickness of the microscaffold) with a quadrilateral base of 0.05 mm x 0.05 mm. The microscaffolds were suspended in phosphate buffer. Microscaffolds with PLGA and PLLA were prepared in a similar way.

The thus prepared microscaffolds in the buffer can be counted using a Nageotte chamber (or other counting chambers) to determine the appropriate concentration of carrier/vehicle for injection or inoculation of cell cultures. In this example, the composition contains 9000 microscaffold/ml buffer.

The said microscaffolds float freely and homogeneously in the volume of the buffer and do not aggregate. Thus, it is possible to inject aqueous suspensions of microscaffolds with needles of small sizes (within the range of 20-27G) without clogging the needle during injection.

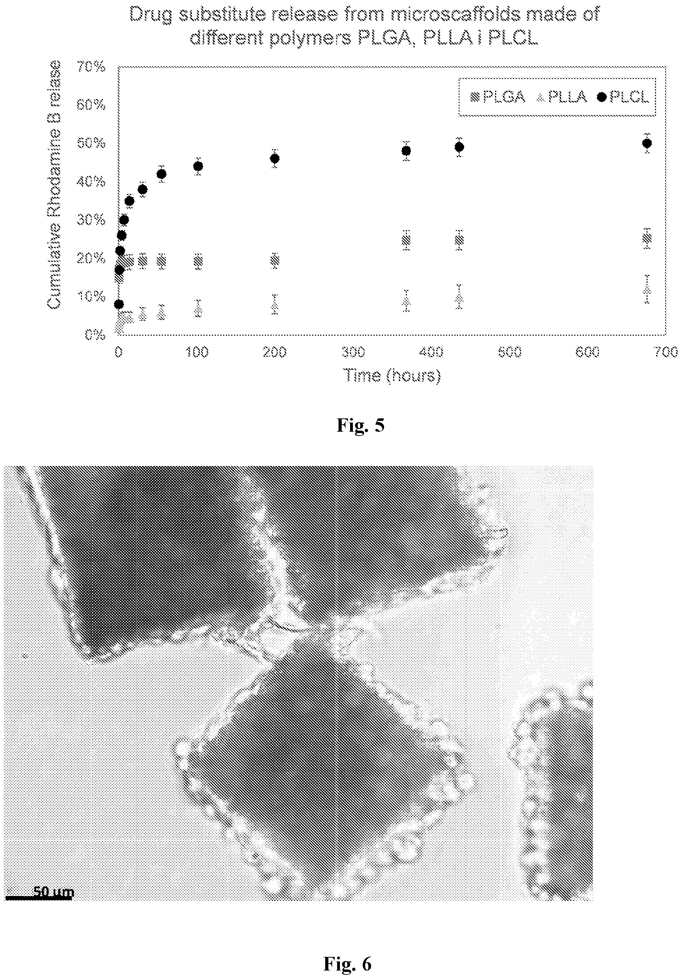

Subsequently, the obtained microscaffolds with PLGA, PLLA, and PLCL were subjected to model substance release analysis (i.e., Rhodamine). The results of the analysis are presented in Fig. 5. As indicated in Fig. 5, the release of the active substance from the microscaffolds contained in the composition according to the invention takes place in a controlled manner, whereby in the first hours after implantation (i.e., injection of the composition according to the invention into the target tissue, e.g., cartilage) there will be an ejection of the drug in the range of 10-40% of the initial dose of the active substance (depending on the polymer used) in the microscaffold, followed by a gradual release of the substance, which lasts for about 2-4 weeks depending on the polymer used.

### Example 16.

### Evaluation of cytotoxicity of microscaffolds fabricated with the method according to the invention.

Cytotoxicity assessment of the material fabricated according to the invention was performed using L929 mouse fibroblasts obtained from the American Type Culture Collection (ATCC). Before the experiment, the microscaffolds were sterilised with 85% ethanol, and after centrifuging and removing the ethanol, the microscaffolds were irradiated with UV light (2 x 30 min).

Mouse L929 fibroblasts, a derivative of Strain L (ATCC^{®} CCL-1^{™}), were cultured according to the manufacturer's instructions (ATTC) using serum-supplemented EMEM (Eagle's Minimum Essential Medium) culture medium (temperature 37°C, atmosphere 95% air, 5% CO₂).

For cytotoxicity analysis, the L929 cells were cultured in non-adherent wells directly in the presence of suspended microscaffolds according to the invention. After 24 h of incubation in an atmosphere with 5% CO₂ and 37°C, live/dead differential staining (Live/Dead Fixable Dead Cell Stains (ThermoFisher Scientific, USA)) was performed. The number and viability of cells, as well as their morphology, were assessed using ActinGreen^{™} and NucBlue^{™} Reagent (Invintrogen^{™}, USA) and fluorescence microscopy.

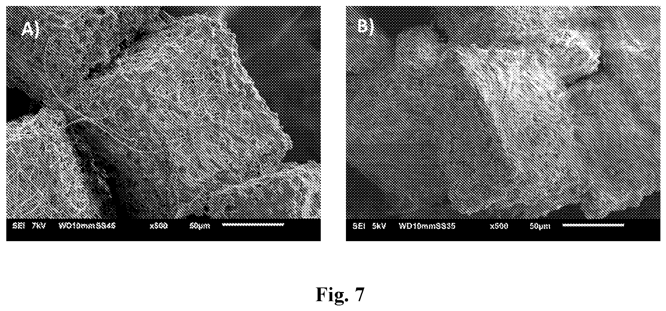

Live cells demonstrated green fluorescence, while dead cells demonstrated red fluorescence. After 24 h of incubation, a large number of live cells and single dead cells were observed. The live cells demonstrated normal morphology and were well spread on the material (Fig. 6 and 7). This observation confirms that the material is not cytotoxic to fibroblasts and promotes cell adhesion.

The lack of cytotoxicity of microscaffolds produced by the method, according to the invention, makes it possible to use them as scaffolds for cells. Such a microscaffold constitutes a porous nonwoven fabric that is a scaffold for cells, which also allows the passage of nutrients, oxygen, and metabolites.

According to the invention, the compositions may thus find use as cellular scaffolds populated *in vitro* with cells taken from patients to replace small bone defects or to regenerate the intervertebral disc in the same patients (patients may, in this case, be cell donors and recipients). Another use of the biomaterial may be to provide a scaffold for cells in bioreactors for expansion of cell cultures.

### Example 17.

### Evaluation of injectability of microscaffolds fabricated with the method according to the invention.

The evaluation of injectability of microscaffolds having a quadrilateral base with dimensions of 0.14 mm by 0.14 mm was performed using 0.5% solution of hyaluronic acid as a vehicle. Fifty microscaffolds in solution were introduced into a 1 mL syringe and then injected through a 23G needle. The same procedure was repeated four times, each time counting the number of microscaffolds coming out of the needle. Tests were also carried out with needle sizes 24G and 26G (Fig. 4).

The obtained results confirm that the composition, according to the invention, can be used as an injectable cell carrier which, due to its small size, can be administered in a minimally invasive manner.

## Claims

1. A method for obtaining an injectable biocompatible drug delivery vehicle, cell carriers or combinations thereof, in the form of microscaffolds, **characterised in that** it comprises the following steps:
a) preparation of a solution comprising a polymer and at least one solvent;
b) fibre formation with a fibre diameter between 50 nm and 10 µm on a flat collector in an electrospinning process;
c) laser cutting of the nonwoven fabric layer formed on the collector into individual detachable microscaffolds with a thickness between 0.01 and 0.2 mm and a cylindrical shape with a base diameter between 0.1 and 0.4 mm or a prism with a base edge length between 0.04 and 0.4 mm;
d) separation of microscaffolds from the collector;
e) chemical modification of the microscaffolds by suspending them in an aqueous NaOH solution of 0.001-1 M for 1-120 min;
f) rinsing excess NaOH from the microscaffold surface.

2. The method according to claim 1, **characterised in that** the polymer is a biodegradable polymer selected from the group consisting of poly(L-lactide), poly(D-lactide), polycaprolactone, poly-(D,L)-lactide, poly-(L-lactide-co-D,L-lactide), polyglycolide, poly(lactide-co-glycolide), poly(lactide-co-caprolactone), polyurethane, chitosan, collagen, mixtures thereof or copolymers.

3. The method according to claim 1 or 2, **characterised in that** in step b), fibres having a diameter of 50 to 1000 nm are formed on the collector.

4. The method according to any of the preceding claims 1 to 3, **characterised in that** in step b) a flat collector selected from the group consisting of a collector made of conductive material, a metal collector, a collector made of thin inorganic material, a glass collector, a collector covered with a layer of conductive material, a collector made of conductive material covered with a polymer film is used.

5. The method according to any of the preceding claims 1 to 4, **characterised in that** in step (a) an active substance is added to the polymer solution in an amount of <1% by weight of pure polymer.

6. The method according to claim 5, **characterised in that** the added active substance is a growth factor selected from the group consisting of BMP-2, BMP-7, TGF, NGF and BDNF.

7. The method according to any of the preceding claims 1 to 6, **characterised in that** in step c) the nanofibre layer is cut with a laser beam selected from the group consisting of an excimer laser, a picosecond laser or a femtosecond laser.

8. The method according to any of the preceding claims 1 to 7, **characterised in that** in step c) the nanofibre layer is cut by a laser beam into prisms with a base of a shape selected from the group consisting of a triangle, square, rectangle, rhombus, parallelogram and trapezoid.

9. The method according to any of the preceding claims 1 to 8, **characterised in that** it comprises a step (g) which includes coating chemically modified, rinsed microscaffolds from step f) with a layer of a substance selected from the group consisting of chitosan, chondroitin sulfate, and growth factor(s).

10. An injectable delivery composition comprising buffer and biocompatible *in vitro* mutually non-aggregating drug vehicles, cell carriers or combinations thereof, **characterised in that** the vehicles are electrospun fibre microscaffolds fabricated according to any of claims 1-9, where each microscaffold is a single vehicle/carrier with a thickness of 0.01 to 0.2 mm and the shape of a cylinder with a base diameter of 0.2 to 0.1 mm or a prism with a base edge length of 0.4 to 0.04 mm, the surface of which is modified chemically with NaOH solution, whereby microscaffolds suspended in the buffer form a suspension of non-aggregating particles.

11. The composition, according to claim 10, **characterised in that** it comprises mammalian cells embedded in microscaffolds, whereby the said mammalian cells are selected from the group consisting of chondrocytes, osteoblasts, fibroblasts and stem cells.

12. The composition according to claim 10 or 11, **characterised in that** the buffer is selected from the group consisting of: buffer containing cell culture medium, phosphate buffer, HEPES buffer (2-[4-(2-hydroxyethyl)-1-piperazinyl]ethanesulfonic acid), MES buffer (2-morpholinoethanesulfonic acid), BIS-TRIS buffer ([bis(2-hydroxyethyl)imino]-tris(hydroxymethyl)methane).

13. The composition according to any of claims 10 to 12, comprising buffer and electrospun microscaffolds which, when suspended in the buffer, form a suspension of non-aggregating microparticles, and each microscaffold is a single vehicle/ carrier with a thickness of 0.1 to 0.2 mm and the shape of a cylinder with a base diameter of 0.01 to 0.2 mm or a prism with a base edge length of 0.1 to 0.4 mm, the surface of which is modified chemically with NaOH solution, for use in the treatment of bone, cartilage or intervertebral disc injuries.

## Patentansprüche

1. Verfahren zur Herstellung eines injizierbaren biokompatiblen Trägers für Arzneimittel, Zellen oder Kombinationen davon in Form von Mikrogerüsten, **dadurch gekennzeichnet, dass** es die folgenden Schritte umfasst:
a) Herstellung einer Lösung, die ein Polymer und mindestens ein Lösungsmittel enthält;
b) Formung von Fasern mit einem Faserdurchmesser zwischen 50 nm und 10 µm auf einem flachen Kollektor durch ein Elektrospinnverfahren;
c) Laserschneiden der auf dem Kollektor gebildeten Vliesschicht in einzelne separate Mikrostrukturen mit einer Dicke zwischen 0,01 und 0,2 mm und entweder einer zylindrischen Form mit einem Durchmesser der Grundfläche von 0,1 bis 0,4 mm oder mit der Form eines Prismas mit einer Seitenlänge der Grundfläche von 0,04 bis 0,4 mm;
d) Trennung der Mikrogerüste vom Kollektor;
e) chemische Modifizierung der Mikrogerüste durch deren Suspendierung in einer wässrigen NaOH-Lösung von 0,001-1 M für 1-120 Min.;
f) Abspülen von überschüssigem NaOH von der Oberfläche der Mikrogerüste.

2. Das Verfahren nach Anspruch 1, **dadurch gekennzeichnet, dass** das Polymer ein biologisch abbaubares Polymer ist, das aus einer Gruppe ausgewählt wird, die Poly(L-Lactid), Poly(D-Lactid), Polycaprolacton, Poly(D,L)-Lactid, Poly(L-Lactid-co-D,L-Lactid), Polyglycolid, Poly(Lactid-co-Glycolid), Poly(Lactid-co-Caprolacton), Polyurethan, Chitosan, Kollagen, deren Mischungen oder Copolymere umfasst.

3. Das Verfahren nach Anspruch 1 oder 2, **dadurch gekennzeichnet, dass** in Schritt (b) Fasern mit einem Durchmesser von 50 bis 1000 nm auf dem Kollektor geformt werden;

4. Verfahren nach einem der vorhergehenden Ansprüche 1 bis 3, **dadurch gekennzeichnet, dass** in Schritt b) ein flacher Kollektor verwendet wird, der aus einer Gruppe ausgewählt wird, die einen Kollektor aus leitfähigem Material, einen Metallkollektor, einen Kollektor aus dünnem anorganischem Material, einen Glaskollektor, einen mit einer Schicht aus leitfähigem Material bedeckten Kollektor und einen mit einem Polymerfilm bedeckten Kollektor aus leitfähigem Material umfasst.

5. Das Verfahren nach einem der vorhergehenden Ansprüche 1 bis 4, **dadurch gekennzeichnet, dass** in Schritt a) der Polymerlösung ein Wirkstoff in einer Menge von < 1 Gew.-% des reinen Polymers zugesetzt wird.

6. Das Verfahren nach Anspruch 5, **dadurch gekennzeichnet, dass** der zugesetzte Wirkstoff ein Wachstumsfaktor ist, der aus einer Gruppe ausgewählt wird, die BMP-2, BMP-7, TGF, NGF oder BDNF umfasst.

7. Das Verfahren nach einem der vorhergehenden Ansprüche 1 bis 6, **dadurch gekennzeichnet, dass** in Schritt c) die Nanofaserschicht mit einem Laserstrahl, ausgewählt aus einer Gruppe, die Excimerlaser, Pikosekundenlaser oder Femtosekundenlaser umfasst, geschnitten wird.

8. Verfahren nach einem der vorhergehenden Ansprüche 1 bis 7, **dadurch gekennzeichnet, dass** in Schritt c) die Nanofaserschicht durch einen Laserstrahl in Prismen geschnitten wird, die in der Basis eine Figur enthalten, die aus einer Gruppe ausgewählt wird, die Dreieck, Quadrat, Rechteck, Raute, Parallelogramm, Trapez umfasst.

9. Verfahren nach einem der vorhergehenden Ansprüche 1 bis 8, **dadurch gekennzeichnet, dass** es einen Schritt g) umfasst, der die Beschichtung der chemisch modifizierten, abgespülten Mikrogerüste aus Schritt f) mit einer Schicht einer Substanz umfasst, die aus einer Gruppe ausgewählt wird, die Chitosan, Chondroitinsulfat, einen Wachstumsfaktor oder Wachstumsfaktoren umfasst.

10. Zusammensetzung zur Verabreichung durch Injektion, die einen Puffer sowie biokompatible, gegenseitig nicht aggregierende *in vitro* Träger für Arzneimittel, Zellen oder Kombinationen davon enthält, **dadurch gekennzeichnet, dass** die Träger Mikrogerüste aus elektrogesponnenen Fasern, hergestellt nach einem der vorhergehenden Ansprüche. 1-9, wobei jedes Mikrogerüst ein einzelner Träger mit einer Dicke von 0,01 bis 0,2 mm und entweder einer zylindrischen Form mit einem Durchmesser der Grundfläche von 0,2 bis 0,1 mm oder mit der Form eines Prismas mit einer Seitenlänge der Grundfläche von 0,4 bis 0,04 mm ist, dessen Oberfläche mit einer NaOH-Lösung chemisch modifiziert ist, wobei die in dem Puffer suspendierten Mikrogerüste eine Suspension aus nicht aggregierenden Mikropartikeln bilden.

11. Die Zusammensetzung nach Anspruch 10, **dadurch gekennzeichnet, dass** sie in Mikrogerüsten eingebettete Säugetierzellen enthält, wobei die Säugetierzellen aus einer Gruppe ausgewählt werden, die Chondrozyten, Osteoblasten, Fibroblasten oder Stammzellen umfasst.

12. Die Zusammensetzung nach Anspruch 10 oder 11, **dadurch gekennzeichnet, dass** der Puffer aus einer Gruppe ausgewählt wird, die Zellkulturmediumpuffer, Phosphatpuffer, HEPES-Puffer (2-[4-(2-Hydroxyethyl)piperazin-1-yl]ethansulfonsäure), MES-Puffer (2-Morpholinoethansulfonsäure), BIS-TRIS-Puffer ([Bis(2-hydroxyethyl)amino]-tris(hydroxymethyl)methan).

13. Die Zusammensetzung nach einem der vorangehenden Ansprüche 10 bis 12, die einen Puffer und elektrogesponnene Mikrogerüste umfasst, die, wenn sie in dem Puffer suspendiert sind, eine Suspension aus nicht aggregierenden Mikropartikeln bilden, wobei jedes Mikrogerüst ein einzelner Träger ist, mit einer Dicke von 0,1 bis 0,2 mm und einer zylindrischen Form mit einem Durchmesser der Grundfläche von 0,01 bis 0,2 mm oder mit der Form eines Prismas mit einer Seitenlänge der Grundfläche von 0,1 bis 0,4 mm, und die Oberfläche jedes Mikrogerüsts chemisch mit einer NaOH-Lösung modifiziert ist, zur Verwendung bei der Behandlung von Knochen-, Knorpel- oder Bandscheibenverletzungen.

## Revendications

1. Procédé d'obtention d'un support biocompatible injectable pour des médicaments, des cellules ou des combinaisons de ceux-ci, sous forme de microstructures, **caractérisé en ce qu'**il comprend les étapes suivantes :
a) préparation d'une solution contenant le polymère et au moins un solvant ;
b) formation de fibres d'un diamètre de 50 nm à 10 µm sur un collecteur plat à l'aide d'un procédé d'électrofilage ;
c) découpe au laser de la couche de non-tissé formée sur le collecteur en microstructures individuelles séparées d'une épaisseur de 0,01 à 0,2 mm et d'une forme cylindrique avec un diamètre de base de 0,1 à 0,4 mm ou d'un prisme avec une longueur de côté de base de 0,04 à 0,4 mm ;
d) séparation des microstructures du collecteur;
e) modification chimique des microstructures en les suspendant dans une solution aqueuse de NaOH de 0,001-1M pendant 1 à 120 minutes ;
f) rinçage de l'excès de NaOH à la surface des microstructures.

2. Procédé selon la revendication 1, **caractérisé en ce que** le polymère est un polymère biodégradable choisi dans le groupe constitué par le poly(L-lactide), le poly(D-lactide), le polycaprolactone, le poly(D,L)-lactide, le poly(L-lactide-ko-D,L-lactide), le polyglycolide, le poly(lactide-co-glycolide), le poly(lactide-co-caprolactone), le polyuréthane, le chitosane, le collagène, leurs mélanges ou les copolymères.

3. Procédé selon la revendication 1 ou 2, **caractérisé en ce qu'**à l'étape b), des fibres d'un diamètre de 50 à 1000 nm sont formées sur le collecteur ;

4. Procédé selon l'une des revendications précédentes 1 à 3, **caractérisé en ce qu'**à l'étape (b) un collecteur plat a été utilisé, choisi dans le groupe constitué par un collecteur en matériau conducteur, un collecteur métallique, un collecteur en matériau inorganique mince, un collecteur en verre, un collecteur recouvert d'une couche de matériau conducteur, un collecteur en matériau conducteur recouvert d'un film de polymère.

5. Procédé selon l'une des revendications précédente 1 à 4, **caractérisé en ce que qu'**à l'étape (a), une substance active est ajoutée à la solution de polymère en une quantité <1 wt% de polymère pur.

6. Procédé selon la revendication 5, **caractérisé en ce que** la substance active ajoutée est un facteur de croissance choisi dans un groupe comprenant BMP-2, BMP-7, TGF, NGF ou BDNF.

7. Procédé selon l'une des revendications précédentes 1 à 6, **caractérisé en ce qu'**à l'étape (c), la couche de nanofibres est découpée à l'aide d'un faisceau laser choisi dans le groupe constitué d'un laser excimer, d'un laser picoseconde ou d'un laser femtoseconde.

8. Procédé selon l'une des revendications précédentes 1 à 7, **caractérisé en ce qu'**à l'étape (c) la couche de nanofibres est découpée par un faisceau laser en prismes contenant à la base une figure choisie dans le groupe comprenant le triangle, le carré, le rectangle, le losange, le parallélogramme, le trapèze.

9. Procédé selon l'une des revendications précédentes 1 à 8, **caractérisée en ce qu'**il comprend une étape (g) consistant à recouvrir les microstructures lavées chimiquement modifiées de l'étape (f) d'une couche d'une substance choisie dans le groupe constitué par le chitosane, le sulfate de chondroïtine, le(s) facteur(s) de croissance.

10. Composition injectable comprenant un tampon et des supports de médicaments, de cellules ou d'une combinaison de ceux-ci, biocompatibles et ne s'agrégeant pas mutuellement in vitro, **caractérisée en ce que** lesdits supports sont des microstructures de fibres électrofilées préparés selon le procédé selon l'une des revendications précédentes 1-9, dans laquelle la microstructure est un support unique ayant une épaisseur de 0,01 à 0,2 mm et une forme cylindrique ayant un diamètre de base de 0,2 à 0,1 mm ou un prisme ayant une longueur de côté de base de 0,4 à 0,04 mm, dont la surface est modifiée chimiquement avec une solution de NaOH, les microstructures en suspension dans le tampon formant une suspension de microparticules non agrégées.

11. Composition selon la revendication 10, **caractérisé en ce qu'** elle contient des cellules de mammifères intégrées dans des microstructures, lesdites cellules de mammifères étant choisies dans un groupe comprenant des chondrocytes, des ostéoblastes, des fibroblastes ou des cellules souches.

12. Composition selon la revendication 10 ou 11, **caractérisé en ce que** le tampon est choisi dans le groupe consistant en : tampon de milieu de culture cellulaire, tampon de phosphate, tampon HEPES (acide 2-[4-(2-hydroxyéthyl)pipérazine-1-yl]éthanesulfonique), tampon MES (acide 2-morpholinoéthanesulfonique), tampon BIS-TRIS ([bis(2-hydroxyéthyl)amino]-tris(hydroxyméthyl)méthane).

13. Composition selon l'une des revendications précédentes 10 à 12, comprenant un tampon et des microstructures électrofilées qui, lorsqu'ils sont en suspension dans le tampon, forment une suspension de microparticules non agrégées, chaque microstructure étant un support unique ayant une épaisseur de 0,1 à 0,2 mm et une forme cylindrique avec un diamètre de base de 0,01 à 0,2 mm ou un prisme dont la longueur du côté de la base est comprise entre 0,1 et 0,4 mm, et la surface de chaque microstructure est modifiée chimiquement par une solution de NaOH, pour le traitement des lésions osseuses, cartilagineuses ou des disques intervertébraux.
